# EUROPEAN PATENT APPLICATION

(11) **EP 1 288 310 A1**
(43) Date of publication of application: **05.03.2003**
(21) Application number: 01120943.4
(22) Date of filing: 31.08.2001
(51) Int. Cl.: C12Q 1/68, C07K 14/72, C12N 15/12, C12N 15/66, C12N 15/70, C12N 15/81

(54) **Use of SNPs of MCH-R for identifying genetic disorders in maintaining the normal body weight**

(71) Applicant: Aventis Pharma Deutschland GmbH, 65929 Frankfurt am Main (DE)
(72) Inventor: Geller, Frank, 58089 Hagen (DE); Hebebrand, Johannes, Prof.Dr., 35039 Marburg (DE); Hinney, Anke, Dr., 35039 Marburg (DE); Wermter, Anne-Kathrin, 35457 Lollar (DE); Ziegler, Andreas, PD Dr., 35457 Lollar (DE)

(57) **Abstract**

A process for identification of a human individuals disposition for a genetic disorder in maintaining this individuals body weight is disclosed.

## Description

The invention refers to a process for identification a human individuals disposition for a genetic disorder in maintaining this individuals normal body weight.

The MCH receptor (MCH-R) is the endogenous receptor for melanin-concentrating hormone. MCH-R is a heptahelical membrane G-protein coupled polypeptide which has previously been designated SCC-1 or GPR 24.
The MCH-R mediates the physiological effect of MCH (Melanin concentrating Hormone) in regulating body weight, metabolism and feeding behavior. MCH is a small, cyclic neuropeptide. It was first isolated from pituitary gland of Salmon, where it functions to regulate scale color. Intracerebral administration of MCH peptide in mammals has been shown to produce a dose dependent stimulation of food intake, whereas mice deficient in MCH exhibit decreased body weight due to reduced feeding behavior and an inappropriately increased metabolic rate. Expression of MCH is increased in the ob mouse model of obesity as well as in normal animals following fasting.

The MCH-receptor with respect to isolated nucleic acids, recombinant host cells and the protein has been disclosed in EP 0 871 669. A splice variant of the MCH-R is described in EP 0 848 060. The sequence of a human MCH-R has first been published by Kolakowski et al., FEBS Lett. 398, 253-258, 1996.
A rat sequence was first disclosed in Lakaye et al., Biochim Biophys Acta, 1401, 216-220, 1998. The human MCH-R sequence is available in public databases (EMBL: AF 008650; NCBI: Z 86090).

However the state of the art offers no possibility to examine whether an individuals inability to control the body weight is due to a genetic disorder.

Therefore the invention refers to a process for identification of a disposition for a genetic disorder in maintaining the normal body weight, wherein
a] polynucleotides are isolated from at least one cell of the individuals body in such a way, that a MCH-receptor gene from that individuals genome is present,
b] the presence or absence of at least one SNP of a MCH-receptor gene is determined from the polynucleotides from a], which SNP is correlated with a genetic disorder in maintaining the normal body weight,
c] and/or the presence or absence of at least one SNP of a MCH-receptor gene is determined from the polynucleotides from a], which SNP is not correlated with a genetic disorder in maintaining the normal body weight,
d] the disposition for a genetic disorder in maintaining the body weight is determined by analysis of the results from b] and/or c].

Preferably the genetic disorders in maintaining the normal body weight results in phenotypic obesitas, body overweight, Anorexia nervosa, bulimia or body under weight.
The polynucleotides shall be isolated in a preferred version of the invention after a tissue sample has been removed from the individuals body. The tissue sample may be cultivated under laboratory conditions before isolation of the polynucleotides takes place. The tissue sample harbors preferably epithelial cells.

Preferably isolation of the polynucleotides could be achieved in vivo or by in vivo techniques.

The presence or absence of a SNP of a MCH-receptor gene will preferably be determined by means of a DNA or RNA molecule which hybridizes under stringent hybridization conditions to a MCH-receptor gene including the non coding regions upstream and downstream within a range of 104b from beginning and of the coding regions or by means of polymerase chain reactions. The sequences of such DNA molecules are given in SEQ ID Nos. 1, 2, 3, 4, 5, 6, 9 or 10.

The SNP used for the process aforementioned is preferably SNP 133073, wherein at position 100 365 of NCBI Z 86090 the C is replaced by a T.

The process of the invention can be used for diagnosis of a genetic disorder in maintaining the normal body weight of a human. The invention refers also to a diagnostic kit containing at least DNA or RNA probes for detection of one or several SNPs of the MCH-receptor gene and/or supplemental compounds as enzymes, buffer substances and/or salts.

The process of the invention can also be used to provide for dietary advice to human individuals with respect to food products and/or intake of food products.

The invention refers also to a polynucleotide comprising complete or part of a MCH-receptor gene sequence wherein at position 100 365 of NCBI Z 86090 the C is replaced by a T. This SNP shall be called SNP 133 073.

The invention refers also to a process for amplification of a polynucleotide comprising the complete or a part of a MCH-receptor gene sequence wherein at position 100 365 of NCBI Z 86090 the C is replaced by a T by first cloning the polynucleotides from human DNA into a cloning vector, and second transforming the cloning vector harboring the said polynucleotide into a microorganism in such a way that the transformed cloning vector will be amplified by the microorganisms. Preferably the microorganism is a bacterial strain of Escherichia coli or a yeast strain of Saccharomyces cerevisiae.

An individuals disposition for a genetic disorder is the individual's receptivity to develop a disease linked to the genetic disorder in dependence on the outer environmental conditions of the individual. Such environmental conditions shall include the location of living, the profession of the individual, his social relationships, the lifestyle and comparable context's. A genetic disorder is a disease caused by a variation or malfunction of the disease underlying genetic constitution. Maintenance of the normal body weight is controlled by genes as for example MCH (Melanin concentrating Hormone), MCH-R (Melanin concentrating Hormone-Receptor), Leptin, the Leptin Receptor or other functions.

The normal body weight of a person can be expressed by the Body mass index (BMI) of this person. The BMI measures the weight/weight ratio.

It is determined by calculating weight in kilograms divided by the square of weight in meters. A normal body mass index is about 19 to 23.

Isolation of polynucleotides can be achieved by using routine techniques. The person skilled in the art will find protocols for such techniques in "F. M. Ansubel et al., Current Protocols in Molecular Biology, Wiley & Sons, New York (currently updated)". The presence of a MCH-receptor gene can be easily detected by means of a polymerase chain reaction using primers as given for example in SEQ ID NOS. 1, 2, 3, 4, 5, 6, 9 or 10. Alternatively the presence of a MCH-receptor gene amongst the polynucleotides isolated can be also determined by blotting the isolated polynucleotides onto a solid matrix as nitrocellulose and hybridizing the blot by homology DNA probes. These protocols will also provide for hybridization's conditions under low medium or high stringency.

In particular a stringent hybridization is carried out by first incubating filters, which carry the polynucleotides to be examined, for 2 hours at 65 °C (in a solution containing 6 x SSPE (52,6 g NaCI, 8,3 g NaH₂PO₄ H₂O, 2,2 g EDTA per liter aqueous solution), 5 x Denhard (10 g Ficoll, 10 g BSA, 10 g Polyvinylpyrrolidine per liter solution) 0,05 % SDS and 100 micrograms tRNA. Thereafter the filters are transferred into a hybridization solution containing a mix as aforementioned with the addition of 10 % Dextran Sulfate and a heat-denatured, radio-labeled DNA probe. The hybridization is carried out for approximately 18 hours at 65°C. The filters are then washed in a solution of 2 x SSC (17,5 g NaCI, 8,8 g Na-Litrat per liter aqueous solution) and 0,5 % SDS at room temperature repeated by a wash in a solution of 0,1 x SSC and 0,1 % SDS at room temperature.

The same techniques as aforementioned (polymerase chain reaction, hybridization) can be also applied for determination of presence or absence of at least one SNP (single nucleotide polymorphism). Whether a SNP of a MCH-receptor is correlated with a genetic disorder has to be analyzed by genetic field experimentation's of risk and non risk populations. Such experimentation's are presented in detail within the example section of this disclosure.

Genetic factors appear to contribute to virtually every human disease, conferring susceptibility or resistance, affecting the severity or progression of disease, and interacting with environmental influences. Much of current biomedical research, in both the public and private sectors, is based upon the expectation that understanding the genetic contribution to disease will revolutionize diagnosis, treatment, and prevention. Defining and understanding the role played by genetic factors in disease will also allow the non-genetic, environmental influence(s) on disease to be more clearly identified and understood.

Analysis of DNA sequence variation is becoming an increasingly important source of information for identifying the genes involved in both disease and in normal biological processes, such as development, aging, and reproducing. In trying to understand disease processes, information about genetic variation is critical for understanding how genes function or malfunction, and for understanding how genetic and functional variation are related. Response to therapies can also be affected by genetic differences. Information about DNA sequence variation will thus have a wide range of application in the analysis of disease and in the development of diagnostic, therapeutic, and preventative strategies.

There are several types of DNA sequence variation, including insertions and deletions, differences in the copy number of repeated sequences, and single base pair differences. The latter are the most frequent. They are termed single nucleotide polymorphisms (SNPs) when the variant sequence type has a frequency of at least 1 % in the population. SNPs have many properties that make them attractive to be the primary analytical reagent for the study of human sequence variation. In addition to their frequency, they are stable, having much lower mutation rates than do repeat sequences. Detection methods of SNPs are potentially more amenable to being automated and used for large-scale genetic analysis. Most importantly, the nucleotide sequence variations that are responsible for the functional changes of interest will often be SNPs.

As noted, SNPs are very common in human DNA. Any two random chromosomes differ at about 1 in 1000 bases. For any particular polymorphic base (i.e., a base where the least common variant has a frequency of at least 1 % in the population), only half or fewer of random pairs of chromosome differ at that site. Thus, there are actually more sites that are polymorphic in the human population, viewed in its entirety, than the number of sites that differ between any particular pair of chromosomes. Altogether, there may be anywhere from 6 million to 30 million nucleotide positions in the genome at which variation can occur in the human population. Thus, overall, approximately one in every 100 to 500 bases in human DNA may be polymorphic.

Information about SNPs will be used in three ways in genetic analysis. First, SNPs can be used as genetic markers in mapping studies. SNPs can be used for whole-genome scans in pedigree-based linkage analysis of families. A map of about 2000 SNPs has the same analytical power for this purpose as a map of 800 microsatellite markers, currently the most frequently used type of marker. Second, when the genetics of a disease are studied in individuals in a population, rather than in families, the haplotype distributions and linkage disequilibria can be used to map genes by association methods. For this purpose, it has been estimated that 30,000 to as many as 300,000 mapped SNPs will be needed.

Third, genetic analysis can be used in case-control studies to directly identify functional SNPs contributing to a particular phenotype. Because only 3-5 % of the human DNA sequence encodes proteins, most SNPs are located outside of coding sequences. But SNPs within protein-coding sequences (which have recently been termed cSNPs) are of particular interest because they are more likely than a random SNPs in non-coding DNA will also have functional consequences, such as those in sequences that regulate gene expression. Discovery of SNPs that affect biological function will become increasingly important over the next several years, and will be greatly facilitated by the availability of a large collection of SNPs, from which candidates for polymorphisms with functional significance can be identified. Accordingly, discovery of a large number of SNPs in human DNA is one objective of this RFA.

SNPs will be particularly important for mapping and discovering the genes associated with common diseases. Many processes and diseases are caused or influenced by complex interactions among multiple genes and environmental factors. These include processes involved in development and aging, and common diseases such as diabetes, cancer, cardiovascular and pulmonary disease, neurological diseases, autoimmune diseases, psychiatric illnesses, alcoholism, common birth defects, disorders maintaining the normal body weight and susceptibility to infectious diseases, teratogens, and environmental agents. Many of the alleles associated with health problems are likely to have low penetrance, meaning that only a few of the individuals carrying them will develop disease. However, because such polymorphisms are likely to be very common in the population, they make a significant contribution to the health burden of the population. Examples of common polymorphisms associated with an increased risk of disease include the ApoE4 allele and Alzheimer's disease, and the APC I1307K allele and colon cancer.

The analysis of the results can be achieved by comparison of the results for presence and absence of SNPs of MCH-R and further assigning the individual to a risk group or not on basis of this comparison which includes determination whether or not a SNP is present and to what extent this SNP is present. The analysis can also refer to statistical methods therein relating for example the linkage of a SNP to a disease to the probability a single individum will be affected by a genetic disorders for maintaining the normal body weight. The analysis can be performed with results from polynucleotides taken from an individual wherein the analysis refers only to the individual person himself or herself. The analysis can be also related to the offspring's of a person when several analysis of different persons will be linked to foresee probability of transfer of the according genetic factors to the following generations.

Obesity is an excess of body fat, frequently resulting in a significant impairment of health. Obesity results when the size or number of fat cells in a person's body increases. A normal sized person has between 30 and 35-10⁷ fat cells. When a person gains weight, these fat cells increase in size at first and later in number.

A normal Body Mass Index (BMI) for adults is about 19 to 23. A BMI of greater than 25 is generally considered overweight. A BMI over 30 is considered obese (World Health Organization). A BMI below 18 is considered underweight.

Individuals with anorexia nervosa are unwilling or unable to maintain a body weight that is normal or expectable for their age and height. The BMI of a person suffering anorexia nervosa in 17.5 or below. Individuals with anorexia nervosa typically display a pronounced fear of weight again and dread of becoming fat although they are dramatically underweight. Concerns and perceptions about their weight have extremely powerful influence and impact on their self evaluation. Diagnostic criteria of anorexia nervosa include two subtypes of the disorder that describe two distinct behavioral patterns. Individuals with the restricting type maintain their low body weight purely by restricting food intake and increased activity. Those with the binge-eating/purging type restrict their food intake but also regularly engage in binge-eating and/or purging behaviors. The syndrome of recurrent episodes of binge-eating is also known as bulimia.

Removal of a tissue sample from an individuals body can be achieved by use of a spatula or spoon therewith scratching epithelial cells off the upper cell layers of the tongue. The tissue can consist of other cell types as liver cells, kidney cells, muscle cells, fat cells, brain cells or other types.

The isolated nucleic acids (e.g. for SNP133073) particularly the DNAs can be introduced into expression vectors or cloning vectors by operatively linking the DNA to the necessary expression control regions (e.g. regulatory regions) required for gene expression or into e.g. multiple cloning site. The vectors can be introduced into the appropriate host cells such as prokaryotic (e.g., bacterial), or eukaryotic (e.g., yeast or mammalian) cells by methods well known in the art (Ausubel et al. supra). The coding sequences for the desired proteins having been prepared or isolated, can be cloned into any suitable vector or replicon. Numerous cloning vectors are known to those of skill in the art, and the selection of an appropriate cloning vector is a matter of choice. Examples of recombinant DNA vectors for cloning and host cells which they can transform include, but is not limited to, the bacteriophage λ (E. coli), pBR322 (E. coli), pACYC177 (E. coli), pKT230 (gram-negative bacteria), pGV1106 (gram-negative bacteria), pLAFR1 (gram-negative bacteria), pME290 (non E. coli gram-negative bacteria), pHV14 (E. coli and Bacillus subtilis), pBD9 (Bacillus), pIJ61 (Streptomyces), pUC6 (Streptomyces), YIp5 (Saccharomyces), a baculovirus insect cell system, a Drosophila insect system, and YCp19 (Saccharomyces). See, generally, "DNA Cloning": Vols. I & II, Glover "Current Protocols in Molecular Biology", Ausubel, F.M., et al. (eds.) Greene Publishing Assoc. and John Wiley Interscience, New York, 1989, 2001).

### Examples

### Study subjects:

We originally screened 215 (127 females) extremely obese German children and adolescents (mean body mass index, BMI 39.78 ± 5.29 kg/m²; mean age 15.27 ± 2.38 years), 230 (110 females) healthy underweight students (mean BMI 18.27 ± 1.10 kg/m²; mean age 25.24 ± 3.72 years), and 91 (85 females) patients who fulfilled lifetime criteria for anorexia nervosa criteria; BMI 16.09 ± 3.36 kg/m²; mean age 18.39 ± 4.93 years) by single strand conformation polymorphism analysis (SSCP) for mutations in exons 1 and 2 of the short form of the human MCH-R. 73 of the AN patients were acutely ill, 18 (16 females) represent patients who where ascertained in a follow-up study. All individuals were independently ascertained and hence are presumably unrelated. BMI of the 215 extremely obese probands all exceeded the 99^{th} BMI-percentile, BMI of underweight students was below the 15^{th} BMI age-percentile.

Based on our initial positive association results (see below) we subsequently screened the first exon of the short form of the human MCH-R in 96 (49 females) healthy normal weight students (mean BMI 21.94 ± 1.06 kg/m²; mean age 24.72 ± 2.58 years) and 97 (50 females) healthy overweight students (mean BMI 29.06 ± 3.43 kg/m²; mean age 25.23 ± 3.67 years) by SSCP in order to detect SNP133073 in the first exon.

Furthermore, to perform the Transmission Disequilibrium Test (TDT) we screened the first exon in both parents of 108 (mean BMI 39.55 ± 5.44 kg/m²; mean age 15.46 ± 2.41 years) of the 215 initially screened extremely obese children and adolescents and in 226 independent trios consisting of an extremely obese children and adolescents (mean BMI 29.14 ± 3.21 kg/m²; mean age 13.04 ± 2.94 years) and both parents to detect SNP133073.

We additionally genotyped the CA-repeat located in the intron (according to 24) in 139 (61 females) underweight students (mean body mass index; BMI 18.41 ± 1.08 kg/m²; mean age 25.46 ± 3.87 years), 122 (67 females) obese children and adolescents (mean BMI 33.37 ± 6.74 kg/m²; mean age 13.68 ± 2.48 years) and in 101 trios consisting of an extremely obese child or adolescent (53 females) (mean BMI 33.08 ± 6.81 kg/m²; mean age 13.53 ± 2.46 years) and both of their parents (99 index patients of these 101 families belonged to the 122 children and adolescents who where genotyped for the CA-repeat; furthermore, the MCHR of 32 index patients of these 101 trios genotyped for the CA-repeat has already been screened by SSCP). Mean BMI and age of the mothers were 30.76 ± 6.27 kg/m² and 40.37 ± 6.55 years, respectively. The corresponding values for the fathers were 29.36 ± 4.61 kg/m² and 43.65 ± 6.74 years.

### SSCP and Sequencing:

PCR was performed with primers flanking exon 1 of the MCH-R: MCH-R-1F (SEQ ID NO.1) 5'GCTCAGCTCGGTTGTGG-3' (100286 - 100 302; NCBI: Z 86090) and MCH-R-amplifying exon 2 of the MCH-R: 1 R 5'GCAGTTTGGCTCAGGGG-3' (SEQ ID NO. 2) (100484 - 100468; NCBI: Z 86090) (199 bp) and primers amplifying exon 2 of the MCH-R: MCH-R-2a-F 5'GCCCATGTCAAACAGCCAAC-3' (SEQ ID NO. 3) (101582 - 101601; NCBI: Z 86090) and MCH-R-2a-R 5'AGGGTGAACCAFTAGAGGTC-3' (SEQ ID NO. 4) (102169 - 102150; NCBI: Z 86090) (588 bp) and MCH-R-2b-F 5'TGCCAGACTCATCCCCT-3' (SEQ ID NO. 5) (102083 - 102099; NCBI: / 86090) and MCH-R-2b-R 5'TTGGAGGTGTGCAGGGT-3' (SEQ ID NO. 6) (102632 - 1026016; NCBI: Z86090) (550 bp) according to standard protocols. Products of MCH-R-2aF/2aR were digested by both Alul (recognition sequence: AG↓CT;

Fermentas, St. Leon Rot, Germany) and Mspl (recognition sequence: C↓CGG Fermentas, St. Leon Rot, Germany) and products of MCH-R-2bF/2bR were digested by Crfl3I (recognition sequence: G/GNCC Fermentas, St. Leon Rot, Germany) prior to SSCP. The digested (exon 2) and the short PCR fragments (exon 1) were diluted in formamide containing buffer and electrophoresed on 21 % acrylamide gels (37.5:1, Q Biogene, Heidelberg, Germany) in 0.5 x TBE buffer (45 mM Tris-HCI; 45 mM Borate and 1.1 mM EDTA). Gels were 16 cm in length and run at two different conditions: a) room temperature for 16h at 400 V and b) 4°C for 17 h at 500 V. Gels were silver stained.

For subsequent sequencing reactions, artificial M13 sequences
(AGGGTTTTCCCAGTCACGACGTT (SEQ ID NO. 7) for the three F-primers, and GAGCGGATAACAATTTCACACAGG (SEQ ID NO. 8) for the three R-primers) were added at the 5' ends of each primer. Bi-directional sequencing of PCR products of all individuals that showed an aberrant SSCP pattern and of two individuals who showed the wild-type SSCP pattern was performed with fluorescently labeled primers (primer sequences complementary to the M13 sequences, F-primers labeled with IRD 700 and R-primers labeled with IRD 800; MWG-Biotech, Ebersberg, Germany). The "Thermo sequenase fluorescent labeled primer cycle sequencing kit with 7-deaza-dGTP" (Amersham, Braunschweig, Germany) was used for cycle-sequencing according to the manufacturer. The sequencing reactions were analyzed on a LiCor 4200-2 automatic sequencer with the Base ImagIR 4.0 software (MWG Biotech, Ebersberg, Germany).

### Microsatellite:

The dinucleotide-repeat (CA-repeat) in the intron of MCH-R (primers flanking the CA-repeat: TTCCAACCAGAGATCTCCAAA-3' ((SEQ ID NO. 9) 101191 - 101211; NCBI:Z86090) and 5'CCAGGAAAACTCGTCAGCAT-3' ((SEQ ID NO. 10) 101319-101300; NCBI: Z86090) was used in an attempt to detect variable alleles in the intron between the two exons of the short form of MCH-R. Genotyping was carried out using fluorescence-based semi-automated technique on an automated DNA sequencing machine (LiCor 4200-2; MWG-Biotech, Ebersberg, FRG). Analyses and assignment of the marker alleles were done with ONE.-Dscan Version 1.3 software (MWG-Biotech).

### Statistical analyzes:

To test for association of the allele frequencies of SNP 133073 to different weight extremes or AN, Pearsons λ² asymptotic two-sided test was used. Additionally, to test for association of the genotype frequencies of this SNP, the Cochran-Armitage Trend asymptotic two-sided test was performed. To test the transmission of the C-allele of SNP133073 the Transmission Disequilibrium Test based on the trios comprising an obese child and both parents was performed.

Furthermore, to test for association of allele frequencies of the dinucleotide-repeat (CA-repeat)in the intron of MCH-R to different weight extremes, Pearsons λ² asymptotic two-sided test was used. Additionally to test for association of the genotype frequencies of this CA-repeat, the Cochran-Armitage Trend asymptotic two-sided test was performed. To test the transmission of the different alleles of the CA-repeat, the Transmission Disequilibrium Test (TDT) based on the 101 trios comprising an obese child and both parents was exploratively performed.

### Analysis:

We initially screened the two excons of the short form of the human MCH-R encoding a 353 AA protein by SSCP in 215 extremely obese children and adolescents, 230 underweight students and in 91 patients with anorexia nervosa. By sequencing of PCR products showing an aberrant SSCP pattern we identified 10 different variations and a single SNP identical to SNP133073 (Table 1).

The mutations are as follows:
(1) A nucleotide exchange (C-100431-T) was detected within the intron in close proximity to the first exon in a single underweight male (BMI 19.53 kg/m², age 23 years).
(2) Within the second exon a total of four silent mutations were detected:
   a) C-101966-T (Tyr-142-Tyr) in a single obese male (BMI 45.63 kg/m², age 24 years).
   b) C-102218-T (Ala-206-Ala) in two obese females (BMI 40.94 kg/m², age 16 years and BMI 34.31 kg/m², age 16 years).
   c) G-102491-A (Thr-297-Thr) in a single obese female (BMI 53.96 kg/m², age 24 years)
   d) G-102515-A (Ser-306-Ser) in a single underweight male (BMI 19.58 kg/m², age 23 years)
(3) Additionally, a total of five missense mutations were detected within this second exon:
   a) G-101962-A (Arg-141-His) in a single underweight male (BMI 19.15 kg/m², age 21 years).
   b) C-102247-T (Thr-216-Met) in a single obese female (BMI 43.22 kg/m², age 13 years).
   c) G-102283-A (Arg-228-Gln) in a single obese female (BMI 43.24 kg/m², age 15 years) and in a single recovered female patient with AN (BMI 18.67 kg/m², age 17 years).
   d) A-102402-C (Thr-268-Pro) in a single underweight female (BMI 16.55 kg/m², age 23 years).
   e) C-102565-T (Thr-322-Met) in two obese probands (female proband: BMI 35.83 kg/m², age 16 years and male proband: BMI 41.33 kg/m², age 15 years) and in one female patient with AN (BMI 16.25 kg/m², age 20 years).

Non of the individuals were homozygous for any of the silent or missense mutations.

In our initial screen we detected the SNP133073 (C-100365-T, Asn-14-Asn) in the first exon of the MCH-R (Table 1). Frequencies of the 100365-C-allele (Table 2, Figure 1) were higher in the obese study group (42 %) than in underweight controls (35 %) and patients with AN (31 %). The allele frequencies (Table 2) differed between the patients with AN and the extremely obese children and adolescents (nominal p = 0.0124) and between the underweight students and the extremely obese children and adolescents (nominal p = 0.0209), but did not differ between patients with AN and the underweight students (nominal p = 0.4327). Genotype frequencies (Table 2) also differed between the extremely obese children and adolescents and the underweight students (nominal p = 0.0159) and the patients with AN (nominal p = 0.0090), respectively, but not differ between the patients with AN and the underweight students (nominal p = 0.4119).

Replication of the association in independent study groups: Based on this initial evidence for association of the C-allele with obesity we screened for the SNP133073 in the first exon of the human MCH-R in 96 healthy normal weight students and in 97 healthy overweight students. In accordance with our hypothesis frequencies of the C-allele (Table 2; Figure 1) were significantly higher in the overweight study group (42 %) as compared to the normal weight study group (31 %; p = 0.0321). Genotype frequencies (Table 2) also different significantly between the overweight students and the normal weight students (p = 0.0238).

In the light of the replicated association indicating an elevated frequency of the C allele of SNP 133073 in obese index patients we subsequently screened the first exon of the human MCH-R by SSCP in a total of 216 parents of a subgroup (n=108) of the 215 extremely obese children and adolescents. Indeed, the TDT revealed a preferential transmission of the C-allele (nominal p = 0.000880).

To replicate this positive TDT the same exon was screened in 226 additional obese children and adolescents and their 452 parents by single strand conformation polymorphism analysis (SSCP) in an attempt to replicate the positive TDT. In accordance with the hypothesized preferential transmission of the C-allele the TDT was significant (p = 0.001219). The allele and genotype frequencies among the 226 additional index patients were very similar to those observed in the two other obese study groups (Table 2). Thus, frequencies of the C-allele (Table 2; Figure 1) were again higher in the 226 obese children of the separate trios (43 %) as compared to the normal weight students (31 %; nominal p = 0.0056) and the underweight students (35 %; nominal p = 0.0096). Genotype frequencies (Table 2) also differed between the 226 obese children of the independent trios and the normal weight students (nominal p = 0.0042) and the underweight students (nominal p = 0.0074), respectively.

The TDT based on both the initial (n=108) and replication (n=226) sample (total number of trios = 334) revealed a p-value of 0.00001. The transmission rates for the C allele were 66.3 % (initial sample), 60 % (replication sample) and 61.9 % (total sample), respectively.

CA-repeat: Genotyping of the CA-repeat in 141 underweight students and 124 obese children and adolescents revealed mainly two alleles: 126 and 128. Frequencies of the 128-allele (Table 3) were somewhat higher in the obese study group (47 %) than in the underweight controls (40 %; p = 0.1156). The genotype frequencies (Table 3) did not differ significantly between the obese and the underweight study groups (p = 0.1067). Two obese children and two underweight individuals with a 130 allele were excluded for the purpose of the test for association.

**Table 1:**

| Mutations and SNP 133073 in the MCH-R in 215 extremely obese children and adolescents, 230 healthy underweight students and 91 patients with anorexia nervosa (AN) | | | | |
|---|---|---|---|---|
| **Study group** | **Base position**^{**+**} | **Effect on amino acid sequence**^{**++**} | **Position within the MCH-R*** | **Frequency of hetero- zygotes#** |
| Extremely obese children and adolescents (n = 215) | C-101966-T | silent | IL 2 | 0.005 |
| | C-102218-T | silent | IL 3 | 0.009 |
| | C-102247-T | Thr-216-Met | IL 3 | 0.005 |
| | G-102283-A | Arg-228-Gln | IL 3 | 0.005 |
| | G-102491-A | silent | C-ter | 0.005 |
| | C-102565-T | Thr-322-Met | C-ter | 0.009 |
| | C-100365-T | silent | N-ter ED | 0.535 |
| Healthy underweight students (n = 230) | C-100431-T | 5' nontranslated region | N-ter ED | 0.005 |
| | G-101962-A | Arg-141-His | IL 2 | 0.005 |
| | A-102402-C | Thr-268-Pro | EL 4 | 0.005 |
| | G-102515-A | silent | C-ter | 0.005 |
| | C-100365-T | silent | N-ter ED | 0.491 |
| Patients with anorexia nervosa (n=91) | G-102283-A | Arg-228-Gln | IL 3 | 0.011 |
| | C-102565-T | Thr-322-Met | C-ter | 0.011 |
| | C-100365-T | silent | N-ter ED | 0.472 |

| | | | | |
|---|---|---|---|---|
| ⁺ See Accession-Nr. NCBI Z 86090 for numbering of genomic sequences. ⁺⁺ See (24) for numbering of amino acid positions. * According to (SWALL:GPRO_HUMAN: http://srs6.ebi.ac.uk/srs6bin/cgi-bin/w...bs%3d {SWALL_SP_REMTREMBL}-prd:AAC14587]. ^{#} Genotype-frequencies are not different from Hardy-Weinberg equilibrium. The polymorphism is shown in shaded boxes. ED: extracellular domain, N-ter: N-terminal, IL:intracellular loop, EL: extracellular loop, C-ter: C-terminal. | | | | |

**Table 2:**

| Allele and genotype frequencies of SNP133073 in the first exon of the human MCH-R in different study groups including 215 extremely obese children and adolescents, 91 patients with anorexia nervosa (AN), 97 healthy overweight, 96 healthy normal weight, 230 healthy underweight students, 108 obese children and adolescents of dependent trios and 226 obese children and adolescents of independent trios, respectively. | | | | | | |
|---|---|---|---|---|---|---|
| **Study group** | **Genotypes**^{**#**} | | | **Alleles** | | |
| | **CC** | **CT** | **TT** | **C-allele** | **T-allele** | **Sum** |
| 1. Patients with AN (n=91) | 7 (7.69 %) | 43 (47.25 %) | 41 (45.05 %) | 57 (31.32 %) | 125 (68.68 %) | 182 |
| 2. Extremely obese children and adolescents (n = 215) | 33 (15.35 %) | 115 (53.49 %) | 67 (31.16 %) | 181 (42.09 %) | 249 (57.91 %) | 430 |
| | | | | | | |
| 2a. Trio subgroup: obese children and adolescents (n = 108) | 18 (16.67 %) | 56 (51.85 %) | 34 (31.48 %) | 92 (42.59 %) | 124 (57.41 %) | 216 |
| 3. Healthy under-weight students (n = 230) | 23 (10.00 %) | 113 (49.13 %) | 94 (40.87 %) | 159 (34.57 %) | 301 (65.43 %) | 460 |
| 4. Healthy normal weight students (n = 96) | 7 (7.29 %) | 46 (47.92 %) | 43 (44.79 %) | 60 (31.25 %) | 132 (68.75 %) | 192 |
| 5. Healthy over-weight students (n = 97) | 14 (14.43 %) | 53 (54.64 %) | 30 (30.93 %) | 81 (41.75 %) | 113 (58.25 %) | 194 |
| 6. Separate trios: obese children and adolescents (n = 226) | 38 (16.81 %) | 118 (52.21 %) | 70 (30.97 %) | 194 (43.11 %) | 258 (56.89 %) | 452 |

| | | | | | | |
|---|---|---|---|---|---|---|
| ^{#} Genotype-frequencies are not different from Hardy-Weinberg equilibrium. nominal p<0.05 for comparisons of allele and genotype frequencies: initial test: 1 versus 2; 2 versus 3 post hoc tests: 3 versus 5,3 versus 6,4 versus 6 significant differences of allele and genotype frequencies (two-sided p<0.05): 4 versus 5 | | | | | | |

**Table 3:**

| Allele and genotype frequencies of the CA-repeat located in the intron of the MCH-R in different study groups including 122 obese children and adolescents and 139 healthy underweight students | | | | | | |
|---|---|---|---|---|---|---|
| **Test for association** | **Genotypes** | | | **Alleles** | | |
| | **126-allele** | **126/128-alleles** | **128-allele** | **126-allele** | **128-allele** | **Sum** |
| Obese children and adolescents (n = 122) | 32 (26.23 %) | 65 (53.28 %) | 25 (20.49 %) | 129 (52.87 %) | 115 (47.13 %) | 244 |
| Healthy underweight students (n = 139) | 48 (34.53 %) | 70 (50.36 %) | 21 (15.11 %) | 166 (59.71 %) | 112 (40.29 %) | 278 |
| ^{#} Genotype-frequencies are not different from Hardy-Weinberg equilibrium. | | | | | | |

(Two obese children and two underweight individuals with a 130 allele were excluded for the purpose of the test for association.)

## Claims

1. Process for identification of a disposition for a genetic disorder in maintaining the normal body weight, wherein
a] polynucleotides are isolated from at least one cell of an individuals body in such a way, that a MCH-receptor gene from that individuals genome is present,
b] the presence or absence of at least one SNP of a MCH-receptor gene is determined from the polynucleotides from a], which SNP is correlated with a genetic disorders in maintaining the normal body weight,
c] and/or the presence or absence of at least one SNP of a MCH-receptor gene is determined from the polynucleotides from a], which SNP is not correlated with a genetic disorder in maintaining the normal body weight,
d] the disposition for a genetic disorder in maintaining the normal body weight is determined by analysis of the results from b] and/or c].

2. A process as claimed in claim 1 wherein the genetic disorder in maintaining the normal body weight results in phenotypic obesitas, body overweight, Anorexia nervosa, bulimia or body underweight.

3. A process as claimed in claim 1 or 2 wherein the isolation of polynucleotides according to step a] takes place after a tissue sample has been removed from an individuals body.

4. A process as claimed in one or several of claims 1 to 3 wherein the tissue sample has been cultivated under laboratory conditions before the polynucleotides will have been isolated.

5. A process as claimed in claims 3 or 4 wherein the tissue sample contains epithelial cells.

6. A process as claimed in one or several of claims 1 to 5 wherein the presence or absence of a SNP of a MCH-receptor gene according to step b] and/or c] is determined by means of a DNA or RNA molecule which hybridizes under stringent hybridization conditions to a MCH-receptor gene including the non-coding regions upstream and downstream within a range of 10 Kb from beginning and end of the coding regions or by means of polymerase chain reactions.

7. A process as claimed in claim 6 wherein the DNA molecule consists of a sequence according to SEQ ID NO. 1, 2, 3, 4, 5, 6, 9 or 10.

8. A process as claimed in claim 1 to 7 wherein the SNP of the MCH-receptor gene is SNP133073, wherein at position 100365 of NCBI Z86090 the C is replaced by a T.

9. Use of a process as claimed in one or several of claims 1 to 8 for diagnosis of a genetic disorder in maintaining the normal body weight of a human individual.

10. Diagnostic kit for use of a process as claimed in claim 9 containing at least DNA or RNA probes for detection of one or several SNP's of the MCH-receptor gene and/or supplemental compounds as enzymes, buffer substances and/or salts.

11. Use of a process as claimed in one or several of claims 1 to 8 for providing dietary advice to human individuals with respect to food products and/or intake of food products.

12. Polynucleotide comprising complete or part of a MCH-receptor gene sequence wherein at position 100365 of NCBI Z86090 the C is replaced by a T (SNP133073).

13. Process for amplification of a polynucleotide as claimed in claim 12 by first cloning the gene sequence from human DNA into a cloning vector and second transforming the cloning vector harboring the polynucleotide sequence into a microorganism wherein the cloning vector will be amplified.

14. Process as claimed in claim 13, wherein the microorganism is a bacterial strain of Escherichia coli or a yeast strain of Saccharomyces cerevisiae.


SEQUENCE LISTING

<110> Aventis Pharma Deutschland GmbH

<120> Use of SNPs of MCH-R for identifying genetic disorders
      in maintaining the normal body weight

<130> AVE D-2001/A042

<140>
<141>

<160> 10

<170> PatentIn Ver. 2.1

<210> 1
<211> 17
<212> DNA
<213> Artificial Sequence

<220>
<223> Description of Artificial Sequence: Part of the
      human MCH-R gene

<400> 1
gctcagctcg gttgtgg                                                17

<210> 2
<211> 17
<212> DNA
<213> Artificial Sequence

<220>
<223> Description of Artificial Sequence: Part of the
      human MCH-R gene

<400> 2
gcagtttggc tcagggg                                                17

<210> 3
<211> 20
<212> DNA
<213> Artificial Sequence

<220>

<223> Description of Artificial Sequence: Part of the
      human MCH-R gene

<400> 3
gcccatgtca aacagccaac                                                    20


<210> 4
<211> 20
<212> DNA
<213> Artificial Sequence

<220>
<223> Description of Artificial Sequence: Part of the
      human MCH-R gene

<400> 4
agggtgaacc agtagaggtc                                                    20


<210> 5
<211> 17
<212> DNA
<213> Artificial Sequence

<220>
<223> Description of Artificial Sequence: Part of the
      human MCH-R gene

<400> 5
tgccagactc atcccct                                                       17


<210> 6
<211> 17
<212> DNA
<213> Artificial Sequence

<220>
<223> Description of Artificial Sequence: Part of the
      human MCH-R gene

<400> 6
ttggaggtgt gcagggt                                                       17


<210> 7
<211> 23

<212> DNA
<213> Artificial Sequence

<220>
<223> Description of Artificial Sequence: Part of M13

<400> 7
agggttttcc cagtcacgac gtt                                              23


<210> 8
<211> 24
<212> DNA
<213> Artificial Sequence

<220>
<223> Description of Artificial Sequence: Part of M13

<400> 8
gagcggataa caatttcaca cagg                                             24


<210> 9
<211> 21
<212> DNA
<213> Artificial Sequence

<220>
<223> Description of Artificial Sequence: Part of the
      human MCH-R gene

<400> 9
ttccaaccag agatctccaa a                                                21


<210> 10
<211> 20
<212> DNA
<213> Artificial Sequence

<220>
<223> Description of Artificial Sequence: Part of the
      human MCH-R gene

<400> 10
ccaggaaaac tcgtcagcat                                                  20